# EUROPÄISCHE PATENTSCHRIFT

(11) **EP 2 658 842 B1**
(45) Veröffentlichungstag und Bekanntmachung des Hinweises auf die Patenterteilung: **12.06.2019**
(21) Anmeldenummer: 11802046.0
(22) Anmeldetag: 15.12.2011
(51) Int. Cl.: C07C 319/20, C07C 319/16

(54) **VERFAHREN ZUR HERSTELLUNG VON METHYLMERCAPTOPROPIONALDEHYD**
PROCESS FOR PREPARING METHYLMERCAPTOPROPIONALDEHYDE
PROCÉDÉ DE PRODUCTION DE MÉTHYLMERCAPTOPROPIONALDÉHYDE

(30) Priorität: 28.12.2010 DE 102010064250
(43) Veröffentlichungstag der Anmeldung: 06.11.2013
(73) Patentinhaber: Evonik Degussa GmbH, 45128 Essen (DE)
(72) Erfinder: FINKELDEI, Caspar Heinrich, 63755 Alzenau (DE); ZACCHI, Pablo, 50354 Hürth (DE); FONFE, Benjamin, 60318 Frankfurt (DE); KRETZ, Stephan, 63599 Biebergemünd (DE); KÖRFER, Martin, 63796 Kahl (DE); HASSELBACH, Hans Joachim, 63571 Gelnhausen (DE); BÖCK, Wolfgang, 63505 Langenselbold (DE)
(74) Vertreter: Evonik Patent Association
(86) Internationale Anmeldenummer: PCT/EP2011/072868
(87) Internationale Veröffentlichungsnummer: WO 2012/089518

(56) Entgegenhaltungen:
- DE-A1- 2 627 430
- US-A- 2 626 282

## Beschreibung

Gegenstand der Erfindung ist ein Verfahren zur Herstellung von Methylmercaptopropionaldehyd (MMP) aus gasförmigem Acrolein (AC) und Methylmercaptan (MC). Gegenstand der vorliegenden Erfindung ist insbesondere ein Verfahren zur Herstellung von Methylmercaptopropionaldehyd aus gasförmigem Acrolein und Methylmercaptan, welches dadurch gekennzeichnet ist, dass in einem Verfahrensschritt gleichzeitig (a) gasförmiges Acrolein von einem Gemisch mindestens enthaltend eine Verbindung aus der Gruppe bestehend aus dem Hemithioacetal des Methylmercaptopropionaldehyds, Methylmercaptopropionaldehyd und Methylmercaptan absorbiert wird, (b) in diesem Gemisch Acrolein mit Methylmercaptan und/oder dem Hemithioacetal des Methylmercaptopropionaldehyds in der Flüssigphase zu Methylmercaptopropionaldehyd umgesetzt wird und (c) Verunreinigungen und Nebenprodukte aus diesem Gemisch entfernt werden, wobei der Verfahrensschritt in einem Reaktivabsorber durchgeführt wird, wobei der Reaktivabsorber dadurch gekennzeichnet ist, dass in nur einem Behälter bzw. in nur einer Stufe gasförmiges Acrolein in einem Gemisch aus hauptsächlich Methlymercaptopropionaldehyd, Methylmercaptan und Hemithioacetal des Methylmercaptopropionaldehyds absorbiert wird, Acrolein direkt bzw. über die Zwischenstufe Hemithioacetal des Methylmercaptopropionaldehyds mit Methylmercaptan zu Methlymercaptopropionaldehyd reagiert und die leichtsiedenden Nebenkomponenten Dimethylsulfid, Acetaldehyd, Wasser und Dimethyldisulfid gestrippt werden.

Die MMP-Bildung aus Methylmercaptan und einem Acrolein enthaltendem Gasgemisch ist aus dem Stand der Technik bekannt.

In DE 2627430 ist beispielsweise ein zweistufiges Verfahren beschrieben, wobei in der ersten Stufe das AC aus einem Gasgemisch in MMP absorbiert wird und in einer zweiten Stufe das in MMP gelöste AC mit MC bei Temperaturen zwischen 10 und 50 °C in Gegenwart eines Katalysators reagiert. Ein großer wirtschaftlicher Nachteil dieser Trennung in zwei Stufen ist die Notwendigkeit der MMP-Rezyklierung bei -10 °C, um AC komplett in MMP zu absorbieren. Die MMP-Ausbeute bezogen auf das in die Absorptionskolonne eingeführte MMP beträgt im beschriebenen Beispiel 99 %. Dabei werden im Reaktionsgemisch vorzugsweise 0,1 bis 0,2 % Hemithioacetal eingestellt. Bei Hemithioacetal-Konzentrationen unter 0,1 % geht AC aufgrund von unvollständigem Umsatz verloren, während sich bei Hemithioacetal-Konzentrationen über 1 % die Ausbeute der MMP-Reaktion verschlechtert. Das während der katalytischen Oxidation von Propylen entstandene Gasgemisch wird von der enthaltenden Acrylsäure in einem Lösungsmittel wie z.B. Tri-n-butylphosphat (FR 7718136), einer Mischung aus Biphenyl und Diphenylether (FR 1393175) oder Wasser (FR 1393175) absorbiert und nach Durchführung dieses Verfahrensschritts in einem Kondensator bei -5 - 0 °C von Wasser befreit. Auch dieser Kondensationsschritt führt zu höheren Investitions- und Betriebskosten.

Gemäß NL-OS 68/09647 ist es auch möglich, zuerst MC mit MMP in der Reaktionszone in Kontakt zu bringen und das so erhaltene Gemisch mit dem AC enthaltenden Gas in Kontakt zu bringen. Allerdings ist hier ein zusätzlicher Schritt zur Behandlung der wässrigen Phase (Extraktion) von Nöten, und es wird nur eine MMP-Ausbeute von 91 % bezogen auf das eingesetzte AC erzielt.

WO 9429254 beschreibt die kontinuierliche Herstellung von MMP aus einem Acrolein enthaltenden Gasgemisch und MC in einer "gas/flüssig"-Reaktionszone, in welcher zusätzlich nicht kondensierbare Gase aus dem AC-Prozess separiert werden. Die Hemithioacetal-Bildung wird durch die äquimolare Zugabe von MC und AC vermieden, überwacht bevorzugt durch periodisch eingesetzte Gaschromatographie. Dadurch soll entsprechend der Beschreibung die MMP-Bildungsgeschwindigkeit um den Faktor 3 - 10 erhöht werden können. Die Limitierung des AC-Stoffübergangs wird durch turbulente Bedingungen im Reaktionssystem klein gehalten.

In allen oben beschriebenen Dokumenten wird destillativ aufgearbeitetes MC eingesetzt. Dies ist ersichtlich daraus, dass die Hauptnebenkomponenten aus der MC-Reaktion, wie Dimethylsulfid und Dimethyldisulfid, weder im MMP-Produkt noch im MMP-Abgas vorhanden sind (WO 9429254 und US 4319047). Die Synthese von MC erfolgt gewöhnlich in der Gasphase bei Temperaturen zwischen 300 und 500 °C und bei Drücken zwischen 1 und 25 bara. Ein Verfahren wird zum Beispiel in der EP 850922 beschrieben. Das Produktgemisch der Synthese enthält neben dem gewünschten MC das bei der Reaktion entstandene Wasser und als Nebenprodukte Dimethylsulfid, Dimethylether, geringe Mengen Polysulfide, sowie nicht umgesetztes Methanol, überschüssigen Schwefelwasserstoff und die im Sinne der Reaktion inerten Gase Stickstoff, Kohlenmonoxid, Kohlendioxid und Wasserstoff. Die Auftrennung des Produktgasgemisches in seine Komponenten dient zur Gewinnung von Methylmercaptan und Dimethylsulfid, zur Ausschleusung von Wasser und Inertgasanteilen sowie zur Rückführung von nicht verbrauchtem Methanol und Schwefelwasserstoff in den Synthesereaktor. Dabei entsteht beispielsweise ein Rein-MC mit einem MC-Gehalt von bis zu 99,6 Gew.-% (EP 0850923 u. DE 1768826). Nachteile dieser destillativen Aufarbeitung des komplexen Reaktionsgemisches sind neben den hohen Investitions- und Betriebskosten die unvermeidliche Bildung von zu entsorgenden Rückständen und damit einhergehend der Verlust von Wertstoffen.

DE 10359636 beschreibt ein Verfahren, welches den hohen destillativen Aufwand zur Gewinnung eines reinen Methylmercaptans vermeidet und trotzdem das bei der katalytischen Umsetzung von H₂S mit Methanol erhaltene Methylmercaptan ohne Verluste für die weitere Umsetzung zu MMP mit flüssigem AC verwendet. Bezogen auf das eingesetzte Roh-MC ist die Isolationsausbeute quasi quantitativ, d.h. > 99,9 %. Dies wird dadurch erreicht, dass die im MMP-Reaktionsgemisch noch enthaltenden Bestandteile aus der MC-Synthese destillativ und bevorzugt durch die Einspeisung eines inerten Schleppmittels, wie z.B. Stickstoff, abgetrennt werden. Das Verfahren ist nicht für den Einsatz von AC enthaltenden Gasen beschrieben.

Aus US 3529940 ist bekannt, dass Reaktionstemperaturen in der MMP-Synthese dadurch kontrolliert werden können, dass die Exothermie der Hemithioacetal-Bildung als Intermediat und nach anschließender Zugabe von Flüssig-AC die MMP-Reaktionsenthalpie der MMP-Reaktion in 2 Zonen aufteilt wird. Allerdings ist das Verfahren ebenfalls nicht für den Einsatz von AC enthaltenden Gasen beschrieben.

Aufgabe der vorliegenden Erfindung ist es daher, in möglichst wenigen Verfahrensschritten aus AC enthaltendem Gas ohne separate Wasserkondensation und aus Roh-MC (MC > 87 Gew.-%, Dimethylsulfid 1,5 - 5 Gew.-%, Dimethyldisulfid 0,2 - 1 Gew.-%, Dimethylether 0 - 3 Gew.-%, Wasser ∼ 0 - 2 Gew.-% und Methanol ∼0 - 2 Gew.-%) MMP von möglichst hoher Reinheit und möglichst hoher Ausbeute bezogen auf die eingesetzten Mengen an AC und MC herzustellen.

Diese Aufgabe wird gelöst durch ein Verfahren zur Herstellung von Methylmercaptopropionaldehyd aus gasförmigem Acrolein und Methylmercaptan, welches dadurch gekennzeichnet ist, dass in einem Verfahrensschritt gleichzeitig (a) gasförmiges Acrolein von einem Gemisch mindestens enthaltend eine Verbindung aus der Gruppe bestehend aus dem Hemithioacetal des Methylmercaptopropionaldehyds, Methylmercaptopropionaldehyd und Methylmercaptan absorbiert wird, (b) in diesem Gemisch Acrolein mit Methylmercaptan und/oder dem Hemithioacetal des Methylmercaptopropionaldehyds in der Flüssigphase zu Methylmercaptopropionaldehyd umgesetzt wird und (c) Verunreinigungen und Nebenprodukte mittels eines Reaktivabsorbers aus diesem Gemisch entfernt werden. Bei der Umsetzung von Acrolein mit Methylmercaptan und/oder Hemithioacetal ist davon auszugehen, dass Hemithioacetal nicht direkt mit Acrolein reagiert, sondern zunächst in Acrolein und Methylmercaptan zerfällt (Gleichgewicht) und das so freigesetzte Methylmercaptan dann mit Acrolein zum MMP reagiert.

Verfahrensschritt (c) wird in einem Reaktivabsorber durchgeführt. Der Reaktivabsorber ist dadurch gekennzeichnet, dass in nur einem Behälter bzw. in nur einer Stufe gasförmiges AC in einem Gemisch aus hauptsächlich MMP, MC und Hemithioacetal absorbiert wird, AC direkt bzw. über die Zwischenstufe Hemithioacetal mit MC zu MMP reagiert und leichtsiedende Nebenkomponenten (u.a. Dimethylsulfid, Acetaldehyd, Wasser und Dimethyldisulfid) gestrippt werden, wobei die Abgasverluste von Edukten und Zielprodukt minimiert werden.

In einer vorteilhaften Ausführungsform enthalten die entfernten Verunreinigungen mindestens eine Verbindung aus der Gruppe bestehend aus Dimethylsulfid, Acetaldehyd, Wasser, Dimethyldisulfid, Methanol, Kohlenstoffdioxid, Propan, Propen, Schwefelwasserstoff und Dimethylether.

Es ist nicht nötig, Wasser aus dem Produkt zu entfernen, um MMP-Ausbeuten bezogen auf das eingesetzte AC in der Nähe von 100 % zu erzielen. In einer bevorzugten Ausführungsform wird Wasser nicht zusätzlich aus dem erhaltenen Methylmercaptopropionaldehyd entfernt.

In der vorliegenden Erfindung wird MMP aus der chemischen Reaktion zwischen AC und MC produziert. Das für diese Reaktion benötigte AC entsteht durch die partielle Oxidation von Propylen in einer Gasphasenreaktion in einem Rohrbündelreaktor. Nach Verlassen des Reaktors wird das AC enthaltende Gas in der Quenchkolonne von überschüssigem Wasser, unerwünschten Nebenprodukten, wie z.B. Essigsäure, Formaldehyd, Allylalkohol, und hauptsächlich Acrylsäure, befreit. Auch ein kleiner Anteil an Acetaldehyd wird an dieser Stelle abgeschieden. Im folgenden Schritt wird AC aus der Gasphase in einer Mischung aus MMP und Hemithioacetal absorbiert und reagiert im gleichen Behälter mit MC bzw. Hemithioacetal.

In einer bevorzugten Ausführungsform wird das für die Reaktion benötigte MC kontinuierlich flüssig oder gasförmig in einen MMP-Zustrom zum Reaktivabsorber dosiert.

Die vorliegende Erfindung ist eine erhebliche Vereinfachung zu oben beschriebenem Stand der Technik, da der eingesetzte Reaktivabsorber zusätzlich als Strippkolonne fungiert und somit zusätzlich zu den leicht siedenden Substanzen aus dem AC enthaltenden Gas auch leicht siedende Nebenkomponenten aus der Reaktion zwischen Schwefelwasserstoff und Methanol, wie z.B. Dimethylsulfid, abtrennt. So ist gewährleistet, dass auch MC mit geringer Reinheit (MC > 87 Gew.-%, Dimethylsulfid 1,5 - 5 Gew.-%, Dimethyldisulfid 0,2 - 1 Gew.-%, Dimethylether 0 - 3 Gew.-%, Wasser ∼ 0 - 2 Gew.-% und Methanol ∼ 0 - 2 Gew.-%) eingesetzt werden kann und somit eine komplexe Aufreinigung, wie beispielsweise in EP 0850923 und DE 1768826 beschrieben, entfallen kann. Es ist bevorzugt, dass das eingesetzte Methylmercaptan 1,5 - 5 Gew.-% Dimethylsulfid und 0 - 3 Gew.-% Dimethylether enthält.

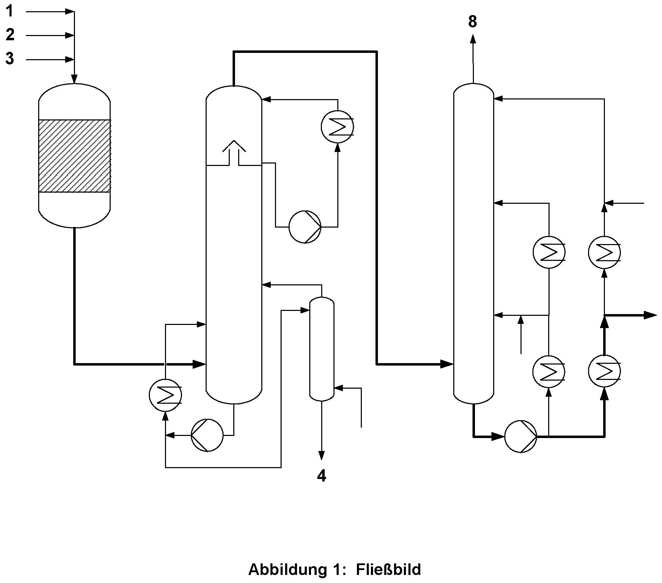

Im folgenden wird die vorliegende Erfindung anhand eines Fließbildes (Abbildung 1) näher beschrieben.

Acrolein wird in einer katalytischen Gasphasenreaktion im Rohrbündelreaktor (A) hergestellt. Am Eingang des Reaktors wird Propylen (1) mit Luft (2) und einem inerten Gasstrom (3) sich zusammensetzend aus Stickstoff und kleinen Mengen an Kohlenstoffdioxid und Wasserdampf vermischt. Die Verdünnung des Reaktionsgases ist von Nöten, um das Risiko einer explosiven Mischung zu vermeiden und um Temperaturspitzen im Katalysatorbett klein zu halten. Die Temperatur der Gasmischung beträgt etwa 130 - 200 °C.

Neben Acrolein und Wasser werden im Rohrbündelreaktor (A) auch Nebenprodukte, wie hauptsächlich Acrylsäure, Essigsäure, Formaldehyd, Acetaldehyd, Kohlenstoffdioxid und Kohlenstoffmonoxid, gebildet. Das Reaktionsgas tritt im unteren Bereich der sogenannten Quenchkolonne (B) ein, in welcher die Temperatur des Gasgemisches durch einen intensiven Kontakt mit Wasser schnell abgekühlt wird. Ein Großteil des Wasserdampfes im Gasgemisch wird kondensiert. In diesem Bereich der Kolonne wird ebenso ein großer Anteil der Nebenprodukte, hauptsächlich Acrylsäure und Essigsäure, zurückgehalten und verlässt die Kolonne (B) über den Sumpf. Diese Flüssigkeit wird über ein sogenanntes "Pumparound"-System umgewälzt und somit als Kühlmedium zum Quenchen des AC-Reaktionsgases verwendet. Auf dem Weg zum Kopf der Kolonne wird das Reaktionsgas im Gegenfluss zu einem Wasserstrom in Kontakt gebracht, was eine weitere Reduzierung des Nebenproduktgehalts im Reaktionsgas bewirkt. Dieser Wasserstrom entsteht aus der Kondensation durch weitere Abkühlung des Reaktionsgases auf < 20 °C (1,2 - 2,5 bara) im oberen Bereich der Kolonne (B) (oberer "Pumparound"). Der die Kolonne (B) verlassende Flüssigstrom kann auf den Kopf einer Strippkolonne (D) gepumpt werden, in welcher der Großteil des gelösten Acroleins wieder gewonnen werden kann. Als Strippmedium (5) können Inertgase eingesetzt werden. Die verbleibende Flüssigkeit (4) kann schließlich einer thermischen Verbrennung zur Entsorgung zugeführt werden.

Vom Kopf der Quenchkolonne (B) tritt das an AC reiche Gas (11) in den Reaktivabsorber (C) im Standardfall über eine Strahlpumpe ein (12). Dies hat den Vorteil, dass Polymerisation von AC vermieden werden kann, eine optimale Druckführung im Reaktivabsorber (C) leichter möglich ist und der vorgeschaltete AC-Prozess bei geringeren Drücken gefahren werden kann. In einer alternativen Ausführungsform kann die AC-Einspeisung auch direkt in die untere Sektion des Reaktivabsorbers erfolgen.

Im Reaktivabsorber (C) wird AC zunächst in einer Mischung aus hauptsächlich MMP, freiem MC, Hemithioacetal und Wasser (12, 13, 14) absorbiert und reagiert dann mit MC bzw. Hemithioacetal in Anwesenheit eines homogenen Katalysators unter Bildung von weiterem MMP. Im Gegensatz zum Stand der Technik ist weder die Hemithioacetal-Bildung aus MC und MMP zu vermeiden (WO 9429254), noch muss MC komplett in Form des Hemithioacetals vor der Reaktion mit AC vorliegen (US 3529940), um quantitative MMP-Ausbeuten bezogen auf das eingesetzte AC zu erhalten. Es ist vorteilhaft, dass das Gemisch das Hemithioacetal des Methylmercaptopropionaldehyds gebildet durch Addition von Methylmercaptan an Methylmercaptopropionaldehyd in einer Konzentration zwischen 0,1 und 10 Gew.-%, bevorzugt zwischen 1 und 10 Gew.-% enthält.

In einer vorteilhaften Ausführungsform wird das Mengenverhältnis zwischen MC und AC in der Flüssigphase des Reaktivabsorbers mit Hilfe einer inline durchgeführten Nahinfrarotmessung (NIR) (Genauigkeit +/- 0,0005 mol/mol) eingestellt. Dabei ist vorteilhaft, dass das Mengenverhältnis zwischen Methylmercaptan und Acrolein in dem Gemisch auf einen Wert zwischen 0,95 und 1,1 mol/mol, bevorzugt zwischen 1,00 und 1,01 mol/mol, besonders bevorzugt zwischen 1,004 und 1,009 mol/mol, und insbesondere bevorzugt auf 1,005 mol/mol eingestellt wird.

In einer bevorzugten Ausführungsform wird die Eingangskonzentration des gasförmigen Acroleins (11) in dem Gemisch mit Hilfe einer inline durchgeführten Fourier transformierten Infrarotmessung (FTIR) erfasst, um die Einspeisung einzustellen, d.h. um die MC-Dosierung (6) möglichst frühzeitig anpassen zu können. Diese inline-Messungen haben den Vorteil, dass unmittelbar auf Änderungen im System reagiert werden kann und damit die Abgasverluste an Edukt und Produkt klein und die MMP-Produktqualität kontinuierlich hoch gehalten werden kann.

Die MMP-Reaktion erfolgt hauptsächlich auf den Einbauten und im Sumpf der Kolonne (C). Als Einbauten können z.B. strukturierte Packungen, regellose Füllkörper oder Böden eingesetzt werden. Der Druck am Kopf der Kolonne (C) liegt im Bereich von 1 bis 2,5 bara, bevorzugt von 1,2 bis 1,6 bara. Eine Pumpe zirkuliert einen Teil des MMPs aus dem Kolonnensumpf durch zwei in Reihe gesetzte Wärmeüberträger, um die Temperatur dieses Stroms zu reduzieren. In der ersten Stufe wird mit "Cooling Tower Water (CTW)" auf ∼ 35 °C gekühlt, und dann mit "Chilled Water (CW)" auf 0 - 20 °C, bevorzugt 5 - 10 °C. Das so abgekühlte MMP tritt zu einem großen Teil am Kopf des Reaktivabsorbers (C) ein (14) und fungiert als Absorptions-/Reaktionsmedium. Ein weiterer Teil des MMPs wird entweder nach dem ersten (bevorzugt) oder dem zweiten Kühlungsschritt als Produktstrom (7) abgeführt. Ein zweiter "Pumparound", der sich im Standardfall an MMP aus dem Sumpf der Kolonne (C) bedient, tritt im mittleren Bereich der Kolonne (C) als Absorptions-/Reaktionsmedium ein (13). Die Temperatur dieses Stroms liegt im Bereich 20 - 50 °C, bevorzugt 30 - 40 °C. Ein in einer besonders bevorzugten Ausführungsform dritter "Pumparound" (12) tritt unterhalb von (13) in die Kolonne ein. Flüssiges und/oder gasförmiges MC (6) wird bevorzugt in diesen Strom zugegeben, kann aber auch an jedem anderen Punkt, bevorzugt im unteren Bereich der Kolonne bzw. in eine der anderen "Pumparounds", zugeführt werden.

Die Temperaturen im unteren Teil der Kolonne können durch den Wärmeüberträger im Strom (12) gezielt gesteuert werden. Die Temperatur in diesem Teil der Kolonne liegt im Bereich 20 °C bis 90 °C, bevorzugt 40 °C bis 75 °C. Die niedrigen Temperaturen am Kopf der Kolonne (C) helfen, AC-, MC- und MMP-Verluste zu minimieren. Allerdings bewirken zu niedrige Temperaturen die Rückhaltung von unerwünschten Komponenten wie Dimethylsulfid (DMS) und Acetaldehyd (AA). Diese Komponenten sind Nebenprodukte der MC- bzw. AC-Reaktion und müssen weitgehend entfernt werden, bevor MMP beispielsweise in der Methionin- oder MHA-Produktion eingesetzt werden kann. Im Standardfall der Erfindung werden diese Nebenprodukte weitgehend im Reaktivabsorber (C) gestrippt und befinden sich im Abgas (8), das die Kolonne (C) am Kopf verlässt und anschließend einer thermischen Verbrennung zugeführt wird.

In einer vorteilhaften Ausführungsform werden die Verunreinigungen und Nebenprodukte durch Strippen bei 0,3 bis 5 bara, bevorzugt bei 1 bis 2 bara, und bei 5 bis 70 °C, bevorzugt bei 5 bis 20 °C, aus dem Gemisch entfernt.

In einer vorteilhaften Ausführungsform wird der Verfahrensschritt in Gegenwart eines homogenen und/oder heterogenen Katalysators (9) durchgeführt. Dabei ist es bevorzugt, dass der Katalysator Dimethylbenzylamin (DMBA) und/oder Triethanolamin ist. Darüber hinaus ist es vorteilhaft, dass die Konzentration des Katalysators in dem Gemisch im Bereich von 50 bis 500 ppmw, bevorzugt im Bereich von 130 bis 150 ppmw liegt.

Zur Regulierung des pH-Werts und der damit verbundenen besseren Lagerstabilität des produzierten MMPs ist es vorteilhaft, dem Gemisch kontinuierlich mindestens eine Verbindung aus der Gruppe bestehend aus anorganischen und organischen Säuren, bevorzugt Essigsäure und Weinsäure, und anorganischen und organischen Basen, bevorzugt Triethanolamin, zuzuführen.

Diese Substanzen bilden beispielsweise im Gemisch mit DMBA die sogenannte Katalysator/Stabilisator-Mischung, welche kontinuierlich dem Reaktivabsorber (C) zugeführt wird. Grundsätzlich sind auch andere Säuren und Basen möglich. In EP 1408029 werden beispielhaft anorganische Oxosäuren, wie z.B. Schwefel- und Phosphorsäure, Halogenwasserstoffe, wie z.B. Wasserstofffluorid, -bromid und -chlorid, genannt. Außerdem sind organische Säuren, wie z.B. aliphatische Monocarbonsäuren (z.B. Ameisensäure, Propionsäure, Octansäure, Acrylsäure, Trichloressigsäure, Trifluoressigsäure), aliphatische Polycarbonsäuren (Oxalsäure, Bernsteinsäure, Adipinsäure), aromatische Monocarbonsäuren (Phenylessigsäure, Benzoesäure, Zimtsäure, Furoesäure, Thiophencarbonsäure) und aromatische Polycarbonsäuren (Phthalsäure, Monoester der Schwefelsäure, Sulfonsäure) geeignet.

Beispiele für basische Substanzen sind anorganische Basen (Ammoniak, Natriumhydroxid, Kaliumhydroxid, Ammoniumcarbonat, Natriumcarbonat, Kaliumcarbonat, Natriumhydrogencarbonat, Kaliumhydrogencarbonat, Ammoniumacetat, Natriumacetat, Kaliumacetat) und stickstoffhaltige organische Basen (Piperidin, Triethylamin, Pyridin, Chinolin, Urotropin, N,N-Dimethylanilin).

Die vorliegende Erfindung hat die Vorteile, dass
▪ die Absorption von AC aus einem Gas, die MMP-Reaktion unter Einsatz von Roh-MC und die Strippung von Leichtsiedern, wie z.B. DMS, DMDS, DME, Methanol, CO₂, H₂S, Wasser, Propen, Propan, Acetaldehyd, in nur einem Verfahrensschritt, beispielsweise in einem Reaktivabsorber, möglich ist,
▪ dass Verluste an MC, AC und MMP minimiert werden können,
▪ dass die MC/AC-Stöchiometrie durch Einsatz eines NIRs und FTIRs kontrolliert werden kann,
▪ dass auf eine separate Wasserkondensation aus dem AC enthaltenden Gas verzichtet werden kann, und
▪ dass die Polymerbildungswahrscheinlichkeit minimiert werden kann.

Die folgenden Ausführungsbeispiele sollen die vorteilhaften Aspekte der vorliegenden Erfindung verdeutlichen.

### 1) Standardfall

Der in den Reaktivabsorber (C) eintretende AC enthaltende Gasstrom (11) hat folgende Zusammensetzung (Tabelle 1):

**Tabelle 1:**

| **Zusammensetzung AC-Prozessgas (Vol-%)** | |
|---|---|
| **CO2** | 0,651 |
| **O2** | 5,112 |
| **Argon** | 0,625 |
| **N2** | 85,147 |
| **H2O** | 1,331 |
| **Propen** | 0,162 |
| **Propan** | 0,030 |
| **CO** | 0,354 |
| **Acetaldehyd** | 0,143 |
| **Acrolein** | 6,430 |
| **Allylalkohol** | 0,003 |
| **Allylacrylat** | 0,001 |
| **Essigsäure** | 0,011 |
| **Acrylsäure** | 0,000 |

Um die zusätzliche Strippwirkung auf Leichtsieder wie Dimethylsulfid und Acetaldehyd zu zeigen, wurde Methylmercaptan (6) in folgender Zusammensetzung (Tabelle 2) in den Reaktivabsorber (C) eingeleitet.

**Tabelle 2:**

| **Zusammensetzung MC (Gew.-%)** | |
|---|---|
| **H2S** | 0,212 |
| **H20** | 0,860 |
| **Dimethylether** | 0,068 |
| **Methanol** | 0,410 |
| **Methylmercaptan** | 95,981 |
| **Dimethylsulfid** | 1,830 |
| **Dimethyldisulfid** | 0,650 |

So findet sich im Abgas (8) der Großteil an Dimethylsulfid und Acetaldehyd wieder, während nur ein sehr geringer Anteil im MMP-Produkt (7) verbleibt. Die MC-, AC- und MMP-Verluste werden sehr gering gehalten (Tabellen 3 und 4).

**Tabelle 3:**

| **Zusammensetzung MMP (Gew.-%)** | |
|---|---|
| **Methylmercaptan** | 0,443 |
| **Acetaldehyd** | 0,043 |
| **Dimethylsulfid** | 0,043 |
| **Acrolein** | 0,015 |
| **Methanol** | 0,126 |
| **H2O** | 2,415 |
| **Dimethyldisulfid** | 0,212 |
| **Allylalkohol** | 0,025 |
| **Allylacrylat** | 0,015 |
| **Dimethylbenzylamin** | 0,016 |
| **MMP** | 96,500 |
| **Acrylsäure** | 0,000 |
| **Essigsäure** | 0,148 |
| **Dimethylether** | 0,000 |

**Tabelle 4:**

| **Zusammensetzung Abgas (Gew.-%)** | |
|---|---|
| **CO2** | 1,064 |
| **H2S** | 0,000 |
| **Wasser** | 0,341 |
| **Propen** | 0,254 |
| **Propan** | 0,049 |
| **Dimethylether** | 0,034 |
| **Methanol** | 0,016 |
| **Acetaldehyd** | 0,223 |
| **Methylmercaptan** | 0,356 |
| **Acrolein** | 0,000 |
| **Dimethylsulfid** | 0,216 |
| **Allylalkohol** | 0,000 |
| **Essigsäure** | 0,014 |
| **Dimethyldisulfid** | 0,021 |
| **Allylacrylat** | 0,000 |
| **MMP** | 0,150 |
| **O2** | 6,079 |
| **CO** | 0,368 |
| **N2** | 89,891 |
| **Argon** | 0,926 |

MMP-Produkt (7) wurde zusätzlich für 25 Minuten bei 200 °C und bei 30 mbara destilliert, um die Rückstandskonzentration zu bestimmen. Diese lag überraschenderweise mit 0,20 - 0,25 Gew.-% sogar unter der Rückstandskonzentration von 0,30 - 0,40 Gew.-% einer MMP-Probe, die aus einem MMP-Verfahren unter Nutzung von Flüssig-AC stammt (US 3529940). Auch war die Rückstandsentwicklung bei Lagerung bis zu 32 Tagen bei der vorliegenden Erfindung mit 0,03 Gew.-% pro Tag geringer als in einem Verfahren unter Nutzung von Flüssig-AC (0,05 Gew.-% pro Tag).

Um die MMP-Qualität für einen nachfolgenden Methioninprozess zu gewährleisten ist also keine separate Wasserkondensation aus dem AC-haltigen Gas (11), wie in DE 2627430 beschrieben, notwendig. Ein gewisser Wasseranteil kann sogar einen positiven Einfluss auf die MMP-Bildungsgeschwindigkeit haben, da Wasser bis zu einer gewissen Konzentration einen höheren Dissoziationsgrad des eingesetzten basischen Aminkatalysators hat. Auch liegen alle Nebenproduktkomponenten im MMP (7) innerhalb der Spezifikation für den nachfolgenden Methioninprozess trotz des Einsatzes des oben beschriebenen Roh-MC's (6). Somit konnte die ausreichende Strippwirkung des Reaktivabsorbers (C) bestätigt werden. Die MC-, AC- und MMP-Verluste im Abgas (8) sind ausreichend klein, um den Prozess wirtschaftlich fahren zu können. Zur möglichst genauen Einstellung der MC/AC-Stöchiometrie von 1,004 - 1,009 eignet sich insbesondere ein NIR-Messgerät. Inline-Messungen erlauben eine schnelle Reaktion auf eine Veränderung im Prozess, wie z.B. eine Anpassung der Feedströme (AC und/oder MC), und damit eine konstant bleibende MMP-Qualität. Zudem werden Edukt- und Produktverluste über das Abgas (8) minimiert. Die Eingangskonzentration des AC-Gases kann über eine online-GC-Messung, aber auch bevorzugt über eine inline-FTIR-Messung erfolgen.

### 2) Einfluss der MC-Einspeisungsstelle im Reaktivabsorber (C)

Bei Einspeisung von MC direkt in die Kolonne (C) ist ein großer Einfluss auf das Reaktionssystem erkennbar. Die erhöhte Menge an MC, die dadurch in der Kolonne absorbiert werden muss, in Verbindung mit exothermer Hemithioacetalbildung resultiert in einem deutlich erhöhten Temperaturprofil im Reaktivabsorber (C). Dieser Effekt führt zusätzlich zu erhöhten MC- und AC-Verlusten im Abgas (8) (Tabelle 5).

**Tabelle 5: Abgasverluste (8) bei unterschiedlichen MC-Einspeisungsstellen**

| | **Standard** | **MC-Dosierung direkt in Kolonne** |
|---|---|---|
| | | |
| **MC** | 0,111 | 0,608 |
| **AC** | 0,030 | 0,281 |
| **MMP** | 0,040 | 0,057 |

## Patentansprüche

1. Verfahren zur Herstellung von Methylmercaptopropionaldehyd aus gasförmigem Acrolein und Methylmercaptan **dadurch gekennzeichnet, dass** in einem Verfahrensschritt gleichzeitig
(a) gasförmiges Acrolein von einem Gemisch mindestens enthaltend eine Verbindung aus der Gruppe bestehend aus dem Hemithioacetal des Methylmercaptopropionaldehyds, Methylmercaptopropionaldehyd und Methylmercaptan absorbiert wird,
(b) in diesem Gemisch Acrolein mit Methylmercaptan und/oder dem Hemithioacetal des Methylmercaptopropionaldehyds in der Flüssigphase zu Methylmercaptopropionaldehyd umgesetzt wird und
(c) Verunreinigungen und Nebenprodukte aus diesem Gemisch entfernt werden, wobei der Verfahrensschritt in einem Reaktivabsorber durchgeführt wird, wobei der Reaktivabsorber **dadurch gekennzeichnet ist, dass** in nur einem Behälter bzw. in nur einer Stufe gasförmiges Acrolein in einem Gemisch aus hauptsächlich Methlymercaptopropionaldehyd, Methylmercaptan und Hemithioacetal des Methylmercaptopropionaldehyds absorbiert wird, Acrolein direkt bzw. über die Zwischenstufe Hemithioacetal des Methylmercaptopropionaldehyds mit Methylmercaptan zu Methlymercaptopropionaldehyd reagiert und die leichtsiedenden Nebenkomponenten Dimethylsulfid, Acetaldehyd, Wasser und Dimethyldisulfid gestrippt werden.

2. Verfahren nach Anspruch 1 **dadurch gekennzeichnet, dass** das Gemisch das Hemithioacetal des Methylmercaptopropionaldehyds gebildet durch Addition von Methylmercaptan an Methylmercaptopropionaldehyd in einer Konzentration zwischen 0,1 und 10 Gew enthält.

3. Verfahren gemäß Anspruch 2 **dadurch gekennzeichnet, dass** das Gemisch zwischen 1 und 10 Gew.-% Hemithioacetal des Methylmercaptopropionaldehyds enthält.

4. Verfahren nach mindestens einem der Ansprüche 1 bis 3 **dadurch gekennzeichnet, dass** die Verunreinigungen und Nebenprodukte durch Strippen bei 0,3 bis 5 bara, bevorzugt bei 1 bis 2 bara, und bei 5 bis 70 °C, bevorzugt bei 5 bis 20 °C, aus dem Gemisch entfernt werden.

5. Verfahren nach mindestens einem der Ansprüche 1 bis 4 **dadurch gekennzeichnet, dass** die entfernten Verunreinigungen mindestens eine Verbindung aus der Gruppe bestehend aus Dimethylsulfid, Acetaldehyd, Wasser, Dimethyldisulfid, Methanol, Kohlenstoffdioxid, Propan, Propen, Schwefelwasserstoff und Dimethylether enthalten.

6. Verfahren nach mindestens einem der Ansprüche 1 bis 5 **dadurch gekennzeichnet, dass** Methylmercaptan kontinuierlich in flüssiger Form und/oder gasförmig dem Gemisch zugeführt wird.

7. Verfahren nach mindestens einem der Ansprüche 1 bis 6 **dadurch gekennzeichnet, dass** der Verfahrensschritt in Gegenwart eines homogenen und/oder heterogenen Katalysators durchgeführt wird.

8. Verfahren nach Anspruch 7 **dadurch gekennzeichnet, dass** der Katalysator Dimethylbenzylamin und/oder Triethanolamin ist.

9. Verfahren nach mindestens einem der Ansprüche 7 bis 8 **dadurch gekennzeichnet, dass** die Konzentration des Katalysators in dem Gemisch im Bereich von 50 bis 500 ppmw, bevorzugt im Bereich von 130 bis 150 ppmw liegt.

10. Verfahren nach mindestens einem der Ansprüche 1 bis 9 **dadurch gekennzeichnet, dass** das Mengenverhältnis zwischen Methylmercaptan und Acrolein in dem Gemisch auf einen Wert zwischen 0,95 und 1,1 mol/mol, bevorzugt zwischen 1,00 und 1,01 mol/mol, besonders bevorzugt zwischen 1,004 und 1,009 mol/mol, und insbesondere bevorzugt auf 1,005 mol/mol eingestellt wird.

11. Verfahren nach Anspruch 10 **dadurch gekennzeichnet, dass** das Mengenverhältnis zwischen Methylmercaptan und Acrolein in dem Gemisch mit Hilfe einer inline durchgeführten Nahinfrarotmessung (NIR) eingestellt wird.

12. Verfahren nach mindestens einem der Ansprüche 1 bis 11 **dadurch gekennzeichnet, dass** die Eingangskonzentration des gasförmigen Acroleins in dem Gemisch mit Hilfe einer inline durchgeführten Fourier transformierten Infrarotmessung (FTIR) erfasst wird, um die Einspeisung einzustellen.

13. Verfahren nach mindestens einem der Ansprüche 1 bis 12 **dadurch gekennzeichnet, dass** dem Gemisch kontinuierlich mindestens eine Verbindung aus der Gruppe bestehend aus anorganischen und organischen Säuren, bevorzugt Essigsäure und Weinsäure, und anorganischen und organischen Basen, bevorzugt Triethanolamin, zugeführt wird.

14. Verfahren nach mindestens einem der Ansprüche 1 bis 13 **dadurch gekennzeichnet, dass** das eingesetzte Methylmercaptan 1,5 - 5 Gew.-% Dimethylsulfid und 0 - 3 Gew.-% Dimethylether enthält.

15. Verfahren nach mindestens einem der Ansprüche 1 bis 14 **dadurch gekennzeichnet, dass** Wasser nicht zusätzlich aus dem erhaltenen Methylmercaptopropionaldehyd entfernt wird.

## Claims

1. Process for preparing methylmercaptopropionaldehyde from gaseous acrolein and methyl mercaptan, **characterized in that**, in one process step, simultaneously,
(a) gaseous acrolein is absorbed from a mixture at least comprising one compound from the group consisting of the hemithioacetal of methylmercaptopropionaldehyde, methylmercaptopropionaldehyde and methyl mercaptan,
(b) in this mixture, acrolein is reacted with methyl mercaptan and/or the hemithioacetal of methylmercaptopropionaldehyde in the liquid phase to give methylmercaptopropionaldehyde and
(c) impurities and by-products are removed from this mixture, wherein the process step is performed in a reactive absorber, wherein the reactive absorber is **characterized in that**, in only one vessel or in only one stage, gaseous acrolein is absorbed in a mixture of principally methylmercaptopropionaldehyde, methyl mercaptan and hemithioacetal of methylmercaptopropionaldehyde, acrolein reacts directly or via the hemithioacetal intermediate of methylmercaptopropionaldehyde with methyl mercaptan to give methylmercaptopropionaldehyde, and the low-boiling secondary components dimethyl sulphide, acetaldehyde, water and dimethyl disulphide are stripped.

2. Process according to Claim 1, **characterized in that** the mixture comprises the hemithioacetal of methylmercaptopropionaldehyde, formed by addition of methyl mercaptan onto methylmercaptopropionaldehyde in a concentration between 0.1 and 10% by weight.

3. Process according to Claim 2, **characterized in that** the mixture comprises between 1 and 10% by weight of the hemithioacetal of mercaptopropionaldehyde.

4. Process according to at least one of Claims 1 to 3, **characterized in that** the impurities and by-products are removed from the mixture by stripping at 0.3 to 5 bara, preferably at 1 to 2 bara, and at 5 to 70°C, preferably at 5 to 20°C.

5. Process according to at least one of Claims 1 to 4, **characterized in that** the impurities removed comprise at least one compound from the group consisting of dimethyl sulphide, acetaldehyde, water, dimethyl disulphide, methanol, carbon dioxide, propane, propene, hydrogen sulphide and dimethyl ether.

6. Process according to at least one of Claims 1 to 5, **characterized in that** methyl mercaptan is supplied to the mixture continuously in liquid and/or gaseous form.

7. Process according to at least one of Claims 1 to 6, **characterized in that** the process step is performed in the presence of a homogeneous and/or heterogeneous catalyst.

8. Process according to Claim 7, **characterized in that** the catalyst is dimethylbenzylamine and/or triethanolamine.

9. Process according to at least one of Claims 7 and 8, **characterized in that** the concentration of the catalyst in the mixture is in the range from 50 to 500 ppmw, preferably in the range from 130 to 150 ppmw.

10. Process according to at least one of Claims 1 to 9, **characterized in that** the ratio between methyl mercaptan and acrolein in the mixture is adjusted to a value between 0.95 and 1.1 mol/mol, preferably between 1.00 and 1.01 mol/mol, more preferably between 1.004 and 1.009 mol/mol, and especially preferably to 1.005 mol/mol.

11. Process according to Claim 10, **characterized in that** the ratio between methyl mercaptan and acrolein in the mixture is adjusted with the aid of an inline near infrared (NIR) measurement.

12. Process according to at least one of Claims 1 to 11, **characterized in that** the input concentration of the gaseous acrolein in the mixture is detected with the aid of an inline Fourier transform infrared (FTIR) measurement in order to adjust the feed rate.

13. Process according to at least one Claims 1 to 12, **characterized in that** at least one compound from the group consisting of inorganic and organic acids, preferably acetic acid and tartaric acid, and inorganic and organic bases, preferably triethanolamine, is supplied continuously to the mixture.

14. Process according to at least one of Claims 1 to 13, **characterized in that** the methyl mercaptan used contains 1.5-5% by weight of dimethyl sulphide and 0-3% by weight of dimethyl ether.

15. Process according to at least one of Claims 1 to 14, **characterized in that** water is not removed additionally from the methylmercaptopropionaldehyde obtained.

## Revendications

1. Procédé de fabrication de méthylmercaptopropionaldéhyde à partir d'acroléine gazeuse et de méthylmercaptan, **caractérisé en ce que**, lors d'une étape de procédé, simultanément
(a) de l'acroléine gazeuse est absorbée par un mélange contenant au moins un composé du groupe constitué par l'hémithioacétal du méthylmercaptopropionaldéhyde, le méthylmercaptopropionaldéhyde et le méthylmercaptan,
(b) l'acroléine est mise en réaction dans ce mélange avec le méthylmercaptan et/ou l'hémithioacétal du méthylmercaptopropionaldéhyde dans la phase liquide pour former le méthylmercaptopropionaldéhyde, et
(c) des impuretés et des produits secondaires sont éliminés de ce mélange, l'étape de procédé étant réalisée dans un absorbeur réactif, l'absorbeur réactif étant **caractérisé en ce que** de l'acroléine gazeuse n'est absorbée dans un mélange constitué principalement de méthylmercaptopropionaldéhyde, de méthylmercaptan et d'hémithioacétal du méthylmercaptopropionaldéhyde que dans une cuve ou qu'à un étage, l'acroléine réagit directement ou par le biais de l'intermédiaire hémithioacétal du méthylmercaptopropionaldéhyde avec le méthylmercaptan pour former le méthylmercaptopropionaldéhyde, et les composants secondaires de point d'ébullition faible sulfure de diméthyle, acétaldéhyde, eau et disulfure de diméthyle sont extraits.

2. Procédé selon la revendication 1, **caractérisé en ce que** le mélange contient l'hémithioacétal du méthylmercaptopropionaldéhyde formé par addition de méthylmercaptan sur le méthylmercaptopropionaldéhyde en une concentration comprise entre 0,1 et 10 % en poids.

3. Procédé selon la revendication 2, **caractérisé en ce que** le mélange contient entre 1 et 10 % en poids d'hémithioacétal du méthylmercaptopropionaldéhyde.

4. Procédé selon au moins l'une quelconque des revendications 1 à 3, **caractérisé en ce que** les impuretés et les produits secondaires sont éliminés du mélange par extraction à une pression de 0,3 à 5 bara, de préférence de 1 à 2 bara, et à une température de 5 à 70 °C, de préférence de 5 à 20 °C.

5. Procédé selon au moins l'une quelconque des revendications 1 à 4, **caractérisé en ce que** les impuretés éliminées contiennent au moins un composé du groupe constitué par le sulfure de diméthyle, l'acétaldéhyde, l'eau, le disulfure de diméthyle, le méthanol, le dioxyde de carbone, le propane, le propène, le sulfure d'hydrogène et l'éther diméthylique.

6. Procédé selon au moins l'une quelconque des revendications 1 à 5, **caractérisé en ce que** le méthylmercaptan est introduit dans le mélange en continu sous forme liquide et/ou gazeuse.

7. Procédé selon au moins l'une quelconque des revendications 1 à 6, **caractérisé en ce que** l'étape de procédé est réalisée en présence d'un catalyseur homogène et/ou hétérogène.

8. Procédé selon la revendication 7, **caractérisé en ce que** le catalyseur est la diméthylbenzylamine et/ou la triéthanolamine.

9. Procédé selon au moins l'une quelconque des revendications 7 à 8, **caractérisé en ce que** la concentration du catalyseur dans le mélange se situe dans la plage allant de 50 à 500 ppm en poids, de préférence dans la plage allant de 130 à 150 ppm en poids.

10. Procédé selon au moins l'une quelconque des revendications 1 à 9, **caractérisé en ce que** le rapport de quantités entre le méthylmercaptan et l'acroléine dans le mélange est ajusté à une valeur comprise entre 0,95 et 1,1 mol/mol, de préférence comprise entre 1,00 et 1,01 mol/mol, de manière particulièrement préférée entre 1,004 et 1,009 mol/mol, et de manière notamment préférée à 1,005 mol/mol.

11. Procédé selon la revendication 10, **caractérisé en ce que** le rapport de quantités entre le méthylmercaptan et l'acroléine dans le mélange est ajusté à l'aide d'une mesure dans le proche infrarouge (NIR) réalisée en ligne.

12. Procédé selon au moins l'une quelconque des revendications 1 à 11, **caractérisé en ce que** la concentration initiale de l'acroléine gazeuse dans le mélange est déterminée à l'aide d'une mesure dans l'infrarouge à transformée de Fourier (FTIR) réalisée en ligne, afin d'ajuster l'alimentation.

13. Procédé selon au moins l'une quelconque des revendications 1 à 12, **caractérisé en ce qu'**au moins un composé du groupe constitué par les acides inorganiques et organiques, de préférence l'acide acétique et l'acide tartrique, et les bases inorganiques et organiques, de préférence la triéthanolamine, est ajouté au mélange en continu.

14. Procédé selon au moins l'une quelconque des revendications 1 à 13, **caractérisé en ce que** le méthylmercaptan utilisé contient 1,5 à 5 % en poids de sulfure de diméthyle et 0 à 3 % en poids d'éther diméthylique.

15. Procédé selon au moins l'une quelconque des revendications 1 à 14, **caractérisé en ce que** l'eau n'est pas éliminée en outre du méthylmercaptopropionaldéhyde obtenu.
